# EUROPÄISCHE PATENTSCHRIFT

(11) **EP 2 468 356 B1**
(45) Veröffentlichungstag und Bekanntmachung des Hinweises auf die Patenterteilung: **21.11.2018**
(21) Anmeldenummer: 11192954.3
(22) Anmeldetag: 12.12.2011
(51) Int. Cl.: A61N 1/08, A61N 1/362, A61N 1/37

(54) **Implantierbares Gerät**
Implantable device
Appareil implantable

(30) Priorität: 21.12.2010 US 201061425249 P
(43) Veröffentlichungstag der Anmeldung: 27.06.2012
(73) Patentinhaber: BIOTRONIK SE & Co. KG, 12359 Berlin (DE)
(72) Erfinder: Weiss, Ingo, 12435 Berlin (DE)
(74) Vertreter: Galander, Marcus

(56) Entgegenhaltungen:
- EP-A1- 1 632 265
- WO-A2-2006/083668
- WO-A2-2008/073445

## Beschreibung

Die Erfindung betrifft ein permanent oder temporär implantierbares Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Auch können solche induzierten Ströme über Elektrodenpole der Elektrodenleitung an umliegendes Gewebe abgegeben werden und so beispielsweise zu unerwünschten Gewebeerwärmungen führen. Deshalb können Herzschrittmacherpatienten heutzutage in der Regel nicht oder nur eingeschränkt in einem Kernspintomografen untersucht werden.

An implantierbaren Herzschrittmachern oder Defibrillatoren (im Folgenden auch gemeinsam als Herzstimulatoren oder IPG (implantable pulse generator) bezeichnet) sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Die PCT-Anmeldung WO2008073445A2 beschreibt eine zum Einsatz in einem Kernspintomografen geeignete Elektrodenleitung.

Der Erfindung liegt die Aufgabe zugrunde, ein Gerät zu schaffen, welches das zuvor beschriebene Problem löst.

Erfindungsgemäß wird diese Aufgabe durch ein temporär oder permanent implantierbares medizinisches Gerät gelöst, das mit wenigstens zwei langgestreckten elektrischen Funktionsleitern für die Übertragung von Therapiesignalen oder Diagnosesignalen oder beidem verbunden oder zu verbinden ist, und wenigstens einem mit wenigstens einem der Funktionsleiter verbundenen Elektrodenpol, über den elektrischer Strom an im Benutzungsfall an umgebendes Körpergewebe abgegeben oder mit dem elektrische Potentiale in im Benutzungsfall umgebenden Gewebe abgefühlt werden können oder beides.

Erfindungsgemäß weist das medizinische Gerät ein Wellentransfermodul auf, das mit dem Funktionsleiter verbunden oder zu verbinden ist und das ausgebildet ist, über einen Funktionsleiter eintreffende Wellen so zu transformieren und als transformierte Wellen auf einen anderen Funktionsleiter oder denselben Funktionsleiter derart gesteuert aufzuschalten, dass sich die Wellen an dem Elektrodenpol destruktiv überlagern.

Ein medizinisches Gerät, für das die Erfindung besonders relevant ist, ist eine Elektrodenleitung z.B. für einen Herzstimulator, bei der die Funktionsleiter elektrische Leiter der Elektrodenleitung sind, wobei die Elektrodenleitung Elektrodenpole aufweist, welche über die Funktionsleiter mit dem Wellentransfermodul elektrisch verbunden sind.

Das medizinische Gerät, beispielsweise ein an eine Elektrodenleitung angeschlossener implantierbarer Herzstimulator, kann ein Gehäuse besitzen, welches elektrisch leitend ausgebildet ist oder einen Elektrodenpol aufweist, über den im Benutzungsfall elektrischer Strom an umgebendes Körpergewebe abgegeben oder mit dem elektrische Potentiale in umgebenden Gewebe abgefühlt werden können oder beides.

Vorzugsweise weist das medizinische Gerät eine Störfelderkennungsvorrichtung auf, die ausgebildet ist, ein Vorhandensein starker elektromagnetischer Felder zu erfassen und im Falle des Vorhandenseins ein entsprechendes Ausgangssignal zu generieren, und die mit einer Steuereinheit zum Steuern des Wellentransfermoduls verbunden ist.

Die Störfelderkennungsvorrichtung kann einen Temperatursensor aufweisen, der so angeordnet ist, dass er eine Erwärmung eines Elektrodenpols erfassen kann. Auf diese Weise erfasst die Störfelderkennungsvorrichtung eine Erwärmung als Folge hochfrequenter Störfelder und somit Störfelder indirekt über ihre Wirkung. Die Temperatursensoren befinden sich an den Elektrodenpolen oder anderen Stellen, die sich infolge der Wechselwirkungen mit elektromagnetischen Feldern erwärmen können.

Alternativ oder zusätzlich kann die Störfelderkennungsvorrichtung einen Sensor für in einen Funktionsleiter induzierte Ströme oder Spannungen aufweisen und somit dazu ausgebildet sein, induzierte Ströme oder Spannungen direkt zu erfassen.

Das Wellentransfermodul weist eine Verzögerungsleitung auf, die elektromagnetische Wellen verzögert und oder dämpft, so dass im Betriebsfall eine transformierte Welle entsteht, die sich mit einer induzierten Welle auf demselben oder einem anderen Funktionsleiter destruktiv überlagert und die induzierte Welle auf diese Weise kompensiert. Die Verzögerungsleitung kann eine Impedanz aufweisen, die diesen Effekt bewirkt.

Gemäß einer Ausführungsvariante ist die Verzögerungsleitung durch eines oder mehrere, vorzugsweise diskrete elektronische Bauelemente aus einer Gruppe realisiert ist, die Spulen, Kondensatoren, ohmsche Widerstände und Übertrager (Impulstransformator) umfasst. Diese bilden beispielsweise eine LC Schaltung, ggf. auch mit einem ohmschen Widerstand, um die Dämpfung einzustellen.

Zusätzlich oder alternativ kann die Verzögerungsleitung räumliche Strukturen aufweisen, die aufgrund ihrer physikalischen Eigenschaften wellenverzögernde und/oder dämpfende Wirkung haben. Solche räumlichen Strukturen sind nicht unbedingt diskrete elektronische Bauteile sondern z.B. ein Wellenleiter, eine Koaxleitung, eine Strip-Line, mit ggf. verlustbehafteten Materialien um die Dämpfung einzustellen. Hierbei ist das Wellentransfermodul vorzugsweise hinsichtlich seiner Wirkung, insbesondere hinsichtlich seiner Verzögerungswirkung und/oder seiner Dämpfungswirkung einstellbar, z.B. indem die einstellbaren Elemente elektrisch, mechanisch, optisch etc bedient werden. Die Einstellung der steuerbaren Verzögerungsleitungen kann auch durch eine externe Programmiereinrichtung erfolgen.

Sinnvollerweise weist das Wellentransfermodul eine Schalteinheit auf, die angeordnet und ausgebildet ist, transformierte Wellen auf einen durch die Schalteinheit bestimmten Funktionsleiter aufzuschalten oder nicht. Dies erlaubt es, das Wellentransfermodul nur zu nutzen, wenn es erforderlich ist und darüber hinaus an den jeweiligen Anwendungsfall anzupassen. Die Einstellung der Schalteinheit kann auch durch eine externe Programmiereinrichtung erfolgen.

Gemäß einer bevorzugten Ausführungsvariante verbindet eine Verzögerungsleitung des Wellentransfermoduls mindestens zwei Funktionsleiter, wobei die Verbindung über die Schalteinheit herzustellen ist. Die Schalteinheit kann die Form einer Schaltmatrix haben, bei der jeder Kreuzpunkt in der Schaltmatrix durch einen Schalter belegt ist.

Gemäß einer weiteren Ausführungsform kann das medizinische Gerät eine Abschlussimpedanzeinheit aufweisen und das Wellentransfermodul eine Schalteinheit aufweisen, die angeordnet und ausgebildet ist, einen Funktionsleiter mit einer Abschlussimpedanzeinheit zu verbinden. Die Abschlussimpedanz kann eine Phasenverschiebung einer Welle auf einem Funktionsleiter bewirken und so ebenfalls zum gewünschten Effekt einer Wellenauslöschung beitragen. Hierzu ist vorzugsweise wenigstens ein Impedanzwert der Abschlussimpedanzeinheit elektrisch, mechanisch oder optisch steuerbar. Die Abschlussimpedanzen können diskret oder physikalisch realisiert sein. Die Einstellung der steuerbaren Abschlussimpedanzen kann auch durch eine externe Programmiereinrichtung erfolgen.

Die Steuereinheit ist vorzugsweise dazu ausgebildet, die steuerbaren Verzögerungsleitungen und/oder der steuerbaren Abschlussimpedanzen und/oder die Schalteinheit in Abhängigkeit eines Ausgangssignals der Störfelderkennungeinrichtung so zu steuern, dass im Einsatzfall eine durch Störfelder induzierte Erwärmung an den Elektrodenpolen minimiert wird.

Besonders bevorzugt ist ein batteriebetriebenes elektronisches Implantat mit einem elektrisch leitfähigen Gehäuse oder einem Gehäuse mit mindestens einem Elektrodenpol, elektrischen Funktionsleitern, die Elektrodenpole elektrischen Durchführungen des Implantats verbinden, um elektrische Signale von dem jeweiligen Elektrodenpol ins Implantat leiten zu können, einer Störfelderkennungsvorrichtung zum Erfassen des Vorhandenseins starker elektro-magnetischer Felder insbesondere MRI Felder, und einem Wellentransfermodul, das von der Störfelderkennungsvorrichtung über eine Steuervorrichtung gesteuert wird und das über mindestens einen Funktionsleiter ins Implantat eintreffende Wellen so transformiert und auf wenigstens einen anderen Funktionsleiter aufschaltet, dass sich die Wellen an den distalen Elektrodenpolen destruktiv überlagern, so dass dort die MRT bedingte Erwärmung minimiert wird.

Alternativ zu einer steuerbaren Abschlussimpedanz kann auch ein steuerbarer Generator vorgesehen sein, der ausgebildet ist, aktiv ein Kompensationssignal in einen Funktionsleiter einzuspeisen. Das einzuspeisende Kompensationssignal besitzt die gleiche Frequenz wie die das Störsignal und eine bestimmte Phasenlage wobei die Frequenz über die Störfelderkennungseinheit ermittelt wird und die Phasenlage und Amplitude nach Auswertung der Temperatursignale so eingestellt werden, dass die Störfeld induzierte Erwärmung an den Elektrodenpolen (ggf allg. den Temperaturmesspunkten) minimiert wird.

Die Erfindung soll nun anhand von Ausführungsbeispielen mit Bezug auf die Figuren näher erläutert werden. Die Figuren zeigen Folgendes:
- Fig. 1: zeigt als implantierbare medizinische Geräte einen implantierbaren Herzstimulator 10 und eine daran angeschlossene implantierbare Elektrodenleitung 20.
- Fig. 2: zeigt in stark schematisierter Darstellung den inneren Aufbau eines erfindungsgemäßen Herzsimulators.
- Fig. 3: zeigt in stark schematisierter Darstellung einen Aufbau einer Verzögerungsleitung.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

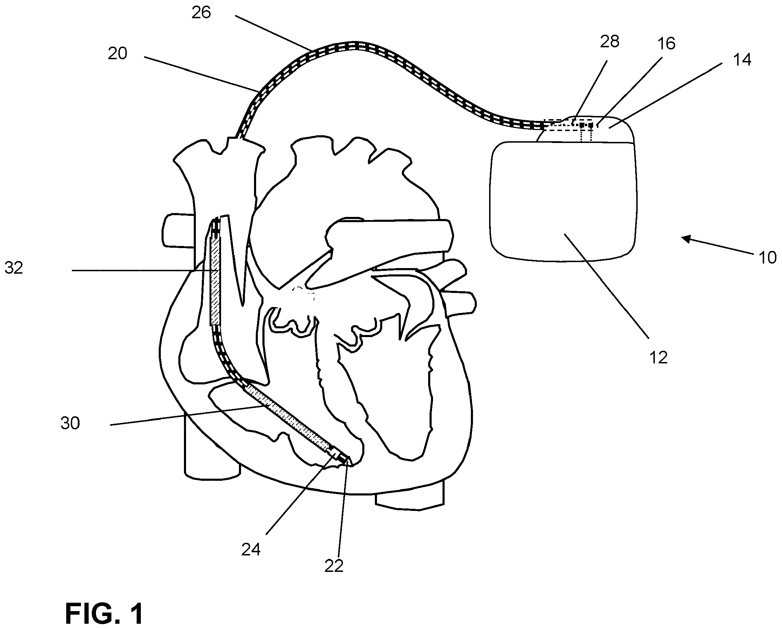

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Figur 1 zeigt, wie sich die Elektrodenpole, also die Tip-Elektrode 22 und die Ringelektrode 24, im Anwendungsfall die Elektrodenleitung 20, im Apex eines rechten Ventrikels eines Herzens befinden.

Sowohl die Tip-Elektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

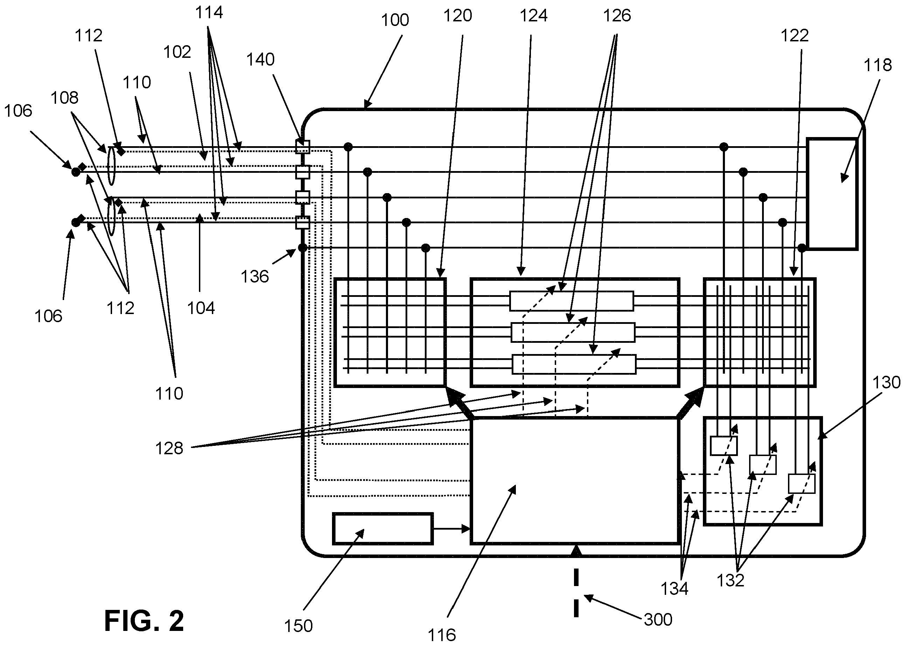

Figur 2 zeigt in schematischer Darstellung den inneren Aufbau eines erfindungsgemäßen medizinischen Gerätes. Das medizinische Gerät besitzt ein elektrisch leitendes Gehäuse 100, das dem Gehäuse 12 aus Figur 1 entspricht. An das Gehäuse angeschlossen sind zwei Elektrodenleitungen 102 und 104, die jeweils eine Tip-Elektrode 106 und eine Ringelektrode 108 aufweisen. Jede der Tip-Elektroden 106 und der Ringelektroden 108 bildet jeweils einen Elektrodenpol. Jeder Elektrodenpol ist über eine separate Zuleitung 110 mit einer Elektronik im Inneren des Gehäuses 100 verbunden. Die Zuleitungen 110 bilden jeweils einen Funktionsleiter. Jeweils in unmittelbarer Nähe der Elektrodenpole 106 und 108 sind Temperatursensoren 112 angeordnet, die über Signalleitungen 114 mit einer Steuereinheit 116 im Inneren des Gehäuses 100 verbunden sind.

Anstelle der Temperatursensoren 112 können auch andere Sensoren zum Erfassen von elektromagnetischen Störfeldern oder von in die Zuleitungen 110 induzierten Strömen oder Spannungen sein.

Die Zuleitungen 110 (Funktionsleiter) und die Signalleitungen 114 sind über in Figur 2 nicht dargestellte Stecker und über Gehäusedurchführungen 140 in das Gehäuse 100 geführt. Die Funktionsleiter 110 steht dabei mit den typischen Bestandteilen eines Herzstimulators sowie Sensingeinheiten oder Stimulationseinheiten in Verbindung. Dies ist in Figur 2 pauschal durch den Block 118 dargestellt, der die sensorische und therapeutische Herzschrittmacherelektronik repräsentiert.

In Figur 2 ist dargestellt, dass die Funktionsleiter 110 einerseits zu der Herzschrittmacherelektronik 118 geführt sind und andererseits zu Schalteinheiten 120 und 122, die als Schaltmatrizen ausgebildet sind. Mit den Schaltmatrizen 120 und 122 ist ein Wellentransfermodul 124 verbunden, das (im abgebildeten Fall drei) einstellbare Verzögerungsleitungen 126 aufweist. Die Verzögerungsleitungen 126 sind hinsichtlich ihrer Verzögerungswirkung und/oder ihrer Dämpfungswirkung einstellbar. Hierzu sind sie über Steuerleitungen 128 mit der Steuereinheit 116 verbunden. Auf diese Weise können sie von der Steuereinheit 116 in Abhängigkeit der Signale empfangen werden, die die Steuereinheit 116 über die Signalleitungen 114 von den Sensoren 112 in den Elektrodenleitungen 102 und 104 empfängt. Konkret ist die Steuereinheit 116 dazu ausgebildet, sowohl die Schaltmatrizen 120 und 122 als auch die Verzögerungsleitungen 126 in Abhängigkeit der über die Signalleitungen 114 empfangenen Signale so zu empfangen, dass die über die Signalleitungen 114 eingehenden Signale möglichst keine Erwärmung der Elektrodenpole 106 und 108 anzeigen. In diesem Sinne kann die Steuereinheit 116 auch als Regler betrachtet werden.

Der letztgenannte Effekt wird mithilfe der einstellbaren Verzögerungsleitungen 126 dadurch erzielt, dass über die Funktionsleiter 110 eingehende Wellen so auf die einstellbaren Verzögerungsleitungen 126 geschaltet werden und die Verzögerungsleitungen 126 so eingestellt werden, dass die Wellen in einer Art und Weise transformiert und auf dieselben oder andere Funktionsleiter geschaltet werden, dass sie sich mit induzierten Wellen destruktiv überlagern und so die Wirkung der induzierten Wellen aufheben.

Diesem Zweck dient alternativ oder zusätzlich auch eine Abschlussimpedanzeinheit 130, die im konkreten Fall drei einstellbare Abschlussimpedanzen 132 aufweist. Die einstellbaren Impedanzen 132 können von der Steuereinheit 116 über Steuerleitungen 134 eingestellt werden. Durch die einstellbaren Abschlussimpedanzen 132 kann die Reflektion der Wellen auf den Funktionsleitern 110 an deren proximalen, durch die Abschlusseinheiten definierten Enden hinsichtlich Phasenlage und Dämpfung eingestellt werden, um auf diese Weise ebenfalls eine destruktive Überlagerung von Wellen im Bereich der Elektrodenpole der jeweiligen Funktionsleiter zu erzielen.

Anstelle der Abschlussimpedanzeinheit 130 kann auch ein Kompensationssignalgenerator vorgesehen sein, der aktiv und durch die Steuereinheit 116 gesteuert Kompensationssignale generiert und in den jeweiligen Funktionsleiter einspeist.

Es sei noch darauf hingewiesen, dass das Gehäuse 100 des Herzstimulators einen eigenen Pol darstellt, der ebenfalls elektrisch (siehe Bezugszeichen 136) mit den Schaltmatrizen 120 und 122 sowie der Herzstimulatorelektronik 118 verbunden ist.

Die Steuereinheit und damit das Verhalten der Schalteinheiten 120 und 122 sowie die Einstellungen der Verzögerungsleitungen 126 und der Abschlussimpedanzen 132 sind von außen programmierbar. Dies ist durch den Pfeil 300 angedeutet.

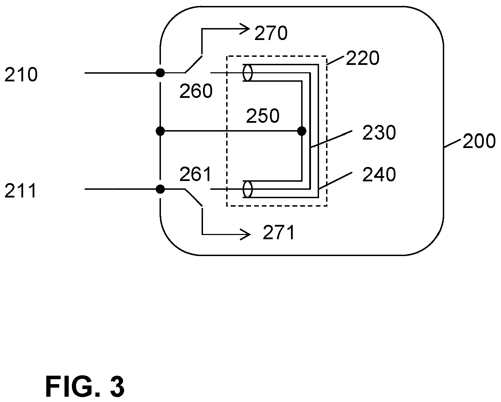

Der Aufbau einer beispielhaften Verzögerungsleitung ist in Figur 3 schematisch dargestellt. In Figur 3 bezeichnen die Bezugszeichen:
- 200:: Leitfähiges Implantatsgehäuse
- 210:: Leitung einer Elektrode
- 211:: Leitung einer Elektrode (derselben Elektrode wie 110 oder einer anderen)
- 220:: Wellentransfermodul
- 230:: Leiter der Verzögerungsleitung
- 240:: Bezugsleiter der Verzögerungsleitung
- 250:: Verbindung des Bezugsleiters der Verzögerungsleitung zum Implantatsgehäuse
- 260, 261:: Schalter
- 270, 271:: Verbindung zur Elektronik

Bei Erkennen eines starken elektromagnetischen Feldes (insbesondere HF Felder wie sie in MR Scannern auftreten und die ein Gefährdungspotential für Patient und Implantat darstellen) durch die Temperatursensoren 112 und die Steuereinheit 116, die in diesem Sinne einer Störfelderkennungseinheit bilden, wird automatisch die Beschaltung der Elektrodeneingänge im Implantat umkonfiguriert. Dies bewirkt die Steuereinheit 116, die entsprechend programmiert oder aufgebaut ist. Diese Beschaltung erfolgt nur temporär während die Störung anliegt, im alltäglichen Betrieb des Implantats wird die hochauflösende und breitbandige Signalaufzeichnung, insbesondere auch die Impedanzermittlung für einen hämodynamischen Sensor, nicht beeinträchtigt. Dann laufen die Elektrodenleitungen direkt (wie herkömmlich) in die Elektronik 118 des Herzstimulators. In einer bevorzugten Realisierung wird während der Störung und erfindungsgemäßen Beschaltung mit Verzögerungsleitungen die Elektronik 118 an einigen oder allen Eingängen weggeschaltet. Ein hierzu vorgesehener, von der Steuereinheit 116 gesteuerter Schalter ist in Figur 2 nicht dargestellt.

In Figur 2 ist eine optionale zusätzliche Störfelderkennungseinheit 150 dargestellt, die auf Störfelder allgemein anspricht und in diesem Fall die Steuereinheit 116, wie zuvor beschrieben, für die Dauer der Störfelder oder eine vorgegebene Zeitdauer aktiviert.

In einer bevorzugten Implementierung erfolgt die Verkopplung der Zuleitungen mittels der Schaltmatrizen 120 und 122 so, dass z.B. der Innenleiter einer ersten Elektrode (koaxial) auf den Außenleiter einer zweiten Elektrode geschaltet wird. In einer weiteren Realisierung wird gleichzeitig der Innenleiter der zweiten Elektrode auf den Außenleiter der ersten Elektrode geschaltet (Kreuzverschaltung). In einer weiteren Realisierung ist dabei die Verzögerung quasi 0 Sekunden, d.h. es wird direkt verschaltet ohne Einsatz von Verzögerungsleitungen.

Bei der Verschaltung der Elektroden wird zusätzlich zur Optimierung hinsichtlich der Erwärmung darauf geachtet, dass die zwischen den Elektroden eingeschlossenen Flächen minimal sind. Damit werden die durch MRI Gradientenfelder induzierte Spannungen minimiert und das Risiko ungewollter (durch den MR Scanner induzierter) Herzstimulation reduziert. Insbesondere wird keine Verbindung zum Gehäusepotential hergestellt.

## Patentansprüche

1. Temporär oder permanent implantierbares medizinisches Gerät (10), mit wenigstens zwei langgestreckten elektrischen Funktionsleitern (110) für die Übertragung von Therapiesignalen oder Diagnosesignalen oder beidem, wobei besagtes Gerät mit den Funktionsleitern verbunden oder zu verbinden ist, und wenigstens einem mit wenigstens einem der Funktionsleiter verbundenen Elektrodenpol (22, 24, 30, 32), über den elektrischer Strom an im Benutzungsfall umgebendes Körpergewebe abgegeben oder mit dem elektrische Potentiale in im Benutzungsfall umgebenden Gewebe abgefühlt werden können oder beides, wobei die Funktionsleiter (110) elektrische Leiter einer Elektrodenleitung (20, 102, 104) sind, die Elektrodenpole aufweist, welche mit den Funktionsleitern (110) elektrisch verbunden sind,
**dadurch gekennzeichnet, dass**
das medizinische Gerät ein Wellentransfermodul (124, 220) aufweist, wobei das Wellentransfermodul (124, 220) eine Verzögerungsleitung (126) aufweist und wobei
das Wellentransfermodul (124, 220) mit den Funktionsleitern (110) verbunden oder zu verbinden ist und das ausgebildet ist, über einen Funktionsleiter (110) eintreffende Wellen so zu transformieren und als transformierte Wellen auf einen anderen Funktionsleiter (110) derart gesteuert aufzuschalten, dass sich die Wellen an dem Elektrodenpol (22, 24, 30, 32) destruktiv überlagern.

2. Medizinisches Gerät nach Anspruch 1, **dadurch gekennzeichnet, dass** das medizinische Gerät 10) ein Gehäuse (12) besitzt, welches elektrisch leitend ausgebildet ist oder einen Elektrodenpol (136) aufweist, über den im Benutzungsfall elektrischer Strom an umgebendes Körpergewebe abgegeben oder mit dem elektrische Potentiale in umgebenden Gewebe abgefühlt werden können oder beides.

3. Medizinisches Gerät nach einem der Ansprüche 1 bis 2, **dadurch gekennzeichnet, dass** das medizinische Gerät (10) eine Störfelderkennungsvorrichtung (150) aufweist, die ausgebildet ist, ein Vorhandensein starker elektromagnetischer Felder zu erfassen und im Falle des Vorhandenseins ein entsprechendes Ausgangssignal zu generieren, und die mit einer Steuereinheit (116) zum Steuern des Wellentransfermoduls (124, 220) verbunden ist.

4. Medizinisches Gerät nach Anspruch 3, **dadurch gekennzeichnet, dass** die Störfelderkennungsvorrichtung (150) einen Temperatursensor aufweist, der so angeordnet ist, dass er eine Erwärmung eines Elektrodenpols (22, 24, 30, 32, 136) erfassen kann.

5. Medizinisches Gerät nach Anspruch 3, **dadurch gekennzeichnet, dass** die Störfelderkennungsvorrichtung (150) einen Sensor für in einen Funktionsleiter (110) induzierte Ströme oder Spannungen aufweist.

6. Medizinisches Gerät nach einem der Ansprüche 1 bis 5, **dadurch gekennzeichnet, dass** die Verzögerungsleitung (126) durch eines oder mehrere elektronische Bauelemente aus einer Gruppe realisiert ist, die Spulen, Kondensatoren, ohmsche Widerstände und Übertrager umfasst.

7. Medizinisches Gerät nach einem der Ansprüche 1 bis 5, **dadurch gekennzeichnet, dass** die Verzögerungsleitung (126) räumliche Strukturen aufweist, die aufgrund ihrer physikalischen Eigenschaften Wellenverzögernde und/oder dämpfende Wirkung haben.

8. Medizinisches Gerät nach einem der Ansprüche 1 bis 7, **dadurch gekennzeichnet, dass** das Wellentransfermodul (124, 220) hinsichtlich seiner Wirkung einstellbar ist.

9. Medizinisches Gerät nach einem der Ansprüche 1 bis 8, **dadurch gekennzeichnet, dass** das Wellentransfermodul (124, 220) eine Schalteinheit (120, 122) aufweist, die angeordnet und ausgebildet ist, transformierte Wellen auf einen durch die Schalteinheit (120, 122) bestimmten Funktionsleiter (110) aufzuschalten oder nicht.

10. Medizinisches Gerät nach Anspruch 3 und einem der Ansprüche 8 bis 9, **dadurch gekennzeichnet, dass** die Steuereinheit (116) ausgebildet ist, die steuerbaren Verzögerungsleitungen (126) und/oder die Schalteinheit (120, 122) in Abhängigkeit eines Ausgangssignals der Störfelderkennungsvorrichtung (150) so zu steuern, dass im Einsatzfall eine durch Störfelder induzierte Erwärmung an den Elektrodenpolen (22, 24, 30, 32, 136) minimiert wird.

## Claims

1. A temporarily or permanently implantable medical device (10) having at least two elongate electrical function conductors (110) for the transmission of therapy signals or diagnostic signals or both, wherein said device is connected or is to be connected to the function conductors, and having at least one electrode terminal (22, 24, 30, 32), which is connected to at least one of the function conductors and via which electrical current can be delivered to surrounding bodily tissue during use or by means of which electrical potentials can be sensed in surrounding tissue during use, or both, wherein the function conductors (110) are electrical conductors of an electrode line (20, 102, 104) which comprises electrode terminals which are electrically connected to the function conductors (110),
**characterised in that**
the medical device comprises a wave transfer module (124, 220), wherein the wave transfer module (124, 220) comprises a delay line (126), and wherein the wave transfer module (124, 220) is connected or is to be connected to the function conductors (110) and is designed to transform waves arriving via one function conductor (110) and to apply them in a controlled manner as transformed waves to another function conductor (110) in such a way that the waves are superimposed destructively at the electrode terminal (22, 24, 30, 32).

2. The medical device according to claim 1, **characterised in that** the medical device (10) has a housing (12) which is electrically conductive or comprises an electrode terminal (136) via which electrical current can be delivered to surrounding bodily tissue during use or by means of which electrical potentials in surrounding tissue can be sensed during use, or both.

3. The medical device according to either claim 1 or 2, **characterised in that** the medical device (10) comprises an interference field recognition apparatus (150) which is designed to detect the presence of strong electromagnetic fields and, if such fields are present, to generate a corresponding output signal, and which is connected to a control unit (116) for controlling the wave transfer module (124, 220).

4. The medical device according to claim 3, **characterised in that** the interference field recognition apparatus (150) comprises a temperature sensor which is arranged such that it can detect a heating of an electrode terminal (22, 24, 30, 32, 136).

5. The medical device according to claim 3, **characterised in that** the interference field recognition apparatus (150) comprises a sensor for currents or voltages induced in a function conductor (110).

6. The medical device according to any one of claims 1 to 5, **characterised in that** the delay line (126) is formed by one or more electronic components from the group comprising coils, capacitors, ohmic resistors and transformers.

7. The medical device according to any one of claims 1 to 5, **characterised in that** the delay line (126) comprises three-dimensional structures which have a wave-delaying and/or damping effect on account of their physical properties.

8. The medical device according to any one of claims 1 to 7, **characterised in that** the wave transfer module (124, 220) is adjustable in respect of its effect.

9. The medical device according to any one of claims 1 to 8, **characterised in that** the wave transfer module (124, 220) comprises a switching unit (120, 122), which is arranged and designed to apply transformed waves to a function conductor (110) determined by the switching unit (120, 122), or not.

10. The medical device according to claim 3 and either one of claims 8 or 9, **characterised in that** the control unit (116) is designed to control the controllable delay lines (126) and/or the switching unit (120, 122) depending on an output signal of the interference field recognition apparatus (150) such that any heating at the electrode terminals (22, 24, 30, 32, 136) induced during use by interference fields is minimised.

## Revendications

1. Appareil médical (10) implantable de manière temporaire ou permanente, doté d'au moins deux conducteurs électriques fonctionnels (110) allongés destinés à la transmission de signaux thérapeutiques ou de signaux de diagnostic, ou des deux, ledit appareil étant relié ou devant être relié avec les conducteurs fonctionnels, et doté d'au moins un pôle d'électrode (22, 24, 30, 32) relié avec au moins un conducteur fonctionnel par le biais duquel un courant électrique peut être délivré à un tissu corporel entourant un lieu d'utilisation, ou à l'aide duquel des potentiels électriques peuvent être détectés dans le tissu entourant le lieu d'utilisation, ou les deux, les conducteurs fonctionnels (110) étant des conducteurs électriques d'une ligne d'électrode (20, 102, 104) qui présente des pôles d'électrode, lesquels sont reliés électriquement avec les conducteurs fonctionnels (110),
**caractérisé en ce que**
l'appareil médical présente un module de transfert d'ondes (124, 220), le module de transfert d'ondes (124, 220) présentant une ligne de délai (126) et le module de transfert d'ondes (124, 220) étant relié ou devant être relié avec les conducteurs fonctionnels (110) et qui est conçu pour transformer des ondes arrivant par le biais d'un conducteur fonctionnel (110) et les brancher sous forme d'ondes transformées sur un autre conducteur fonctionnel (110) de manière à ce que les ondes fassent des interférences destructives au niveau du pôle d'électrode (22, 24, 30, 32).

2. Appareil médical selon la revendication 1, **caractérisé en ce que** l'appareil médical (10) possède un boitier (12), lequel est conçu conducteur électriquement ou présente un pôle d'électrode (136) par lequel du courant électrique peut être appliqué sur du tissu corporel entourant le lieu d'utilisation ou avec lequel des potentiels électriques peuvent être délivrés dans le tissu environnant, ou les deux.

3. Appareil médical selon la revendication 1 à 2, **caractérisé en ce que** l'appareil médical (10) présente un dispositif de reconnaissance de champ brouillage (150), qui est conçu pour détecter la présence de champs électromagnétiques élevés et dans le cas de la présence, générer un signal démission correspondant, et qui est relié avec une unité de commande (116) pour la commande du module de transfert d'ondes (124, 220).

4. Appareil médical selon la revendication 3, **caractérisé en ce que** le dispositif de reconnaissance de champs de brouillage (150) présente un capteur de température qui est disposé de telle manière qu'il peut détecter le réchauffement d'un pôle d'électrode (22, 24, 30, 32, 136).

5. Appareil médical selon la revendication 3, **caractérisé en ce que** le dispositif de reconnaissance de champs de brouillage (150) présente un capteur pour des courants ou des tensions induits dans un conducteur fonctionnel (110).

6. Appareil médical selon l'une des revendications 1 à 5, **caractérisé en ce que** la ligne de délai (126) est réalisée par un ou plusieurs composants électroniques d'un groupe qui comprend des bobines, des condensateurs, des résistances ohmiques et des transmetteurs.

7. Appareil médical selon l'une des revendications 1 à 5, **caractérisé en ce que** la ligne de délai (126) présente des structures dans l'espace qui, en raison de leurs propriétés, ont un effet causant un retard des ondes et/ou un effet d'amortissement.

8. Appareil médical selon l'une des revendications 1 à 7, **caractérisé en ce que** le module de transfert d'ondes (124, 220) peut être réglé au niveau de son action.

9. Appareil médical selon l'une des revendications 1 à 8, **caractérisé en ce que** le module de transfert d'ondes (124, 220) présente une unité de commutation (120, 122) qui est disposée et prévue pour couper ou non des ondes transformées sur un conducteur fonctionnel (110) donné par l'unité de commutation (120, 122).

10. Appareil médical selon la revendication 3, et l'une des revendications 8 à 9, **caractérisé en ce que** l'unité de commande (116) est conçue pour commander les lignes de délai (126) pouvant être commandées et/ou l'unité de commutation (120, 122) en fonction d'un signal d'émission du dispositif de reconnaissance de champs de brouillage (150) de telle manière, qu'en fonctionnement, un réchauffement induit par des champs de brouillage est minimisé au niveau des pôles d'électrode (22, 24, 30, 32, 136).
